# EUROPÄISCHE PATENTANMELDUNG

(11) **EP 2 177 914 A1**
(43) Veröffentlichungstag der Anmeldung: **21.04.2010**
(21) Anmeldenummer: 08166955.8
(22) Anmeldetag: 17.10.2008
(51) Int. Cl.: G01N 35/00

(54) **Testbandeinheit und Testbandgerät**

(71) Anmelder: F. Hoffmann-La Roche AG, 4070 Basel (CH); Roche Diagnostics GmbH, 68305 Mannheim (DE)
(72) Erfinder: Schosnig, Stefan, DE-69493 Hirschberg-Grosssachsen (DE); Heck, Wolfgang, DE-68526 Ladenburg (DE); Porsch, Ulrich, DE-69469 Weinheim (DE); Lorenz, Robert, DE67547 Worms (DE); Kehr, Ulrich, DE-71116 Gärtringen (DE); Treinzen, Andree, DE-71296 Heimsheim (DE); Steinacher, Beda, CH-5430 Wettingen (CH); Sieber, Stefan, CH-8959 Schlieren (CH); Manser, Udo, DE-68723 Schwetzingen (DE); Miltner, Karl, DE-67227 Frankenthal (DE)
(74) Vertreter: Pfiz, Thomas

(57) **Zusammenfassung**

Testbandeinheit mit einem durch einen Bandantrieb (16) vorspulbaren flexiblen Trägerband (34), auf welchem eine Vielzahl von analytischen Testfeldern (32) zur Beaufschlagung mit Körperflüssigkeit in jeweils einem zugehörigen Bandabschnitt (42) aufgebracht sind. Erfindungsgemäß wird vorgeschlagen, dass jeder Bandabschnitt (42) mehrere mittels eines Bandsensors (20) abtastbare und dabei durch eine Messgröße einzeln identifizierbare Positionsmarken (44) für unterschiedliche Funktionsstellungen aufweist.

## Beschreibung

Die Erfindung betrifft eine Testbandeinheit mit einem durch einen Bandantrieb vorspulbaren flexiblen Trägerband, auf welchem eine Vielzahl von analytischen Testfeldern zur Beaufschlagung mit Körperflüssigkeit insbesondere für Glukosetests aufgebracht sind, wobei jedem Testfeld ein Bandabschnitt) zugeordnet ist. Die Erfindung betrifft weiter ein Testbandgerät zur Verarbeitung einer solchen Testbandeinheit.

Eine solche Testbandeinheit ist aus der EP-A 1 739 432 der Anmelder bekannt. Dort sind zwischen den Testfeldern Wegerfassungsbereiche in Form von Rasterstreifen vorgesehen, die während der Bandbewegung zur Erfassung des Bandtransportwegs abgetastet werden, wobei die entsprechenden Signalflankenwechsel in einem Zähler aufsummiert werden, so dass der Zählerstand proportional zum zurückgelegten Bandweg ist. Daraus lässt sich auch eine Positionserfassung ableiten, wobei diesbezüglich nur die schnelle und exakte Positionierung der Testfelder an der vorgesehenen Messstelle angesprochen ist. Die abgetasteten Weginkremente sind allerdings untereinander nicht unterscheidbar, so dass diese mittelbare Art der Positionsbestimmung voraussetzt, dass die Bandstellung nicht unbemerkt verändert wurde.

Ausgehend hiervon liegt der Erfindung die Aufgabe zugrunde, die im Stand der Technik vorgeschlagenen Systeme weiter zu verbessern und eine erhöhte Benutzerfreundlichkeit und Funktionalität im Messablauf zu gewährleisten.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, durch eine Bandcodestruktur unterschiedliche Funktionsstellungen für jeden Test eindeutig zu definieren. Dementsprechend wird erfindungsgemäß vorgeschlagen, dass jeder Bandabschnitt mehrere mittels eines Bandsensors abtastbare und dabei durch eine Messgröße einzeln identifizierbare Positionsmarken für unterschiedliche Funktionsstellungen aufweist. Auf diese Weise kann eine genaue und robuste Bandpositionierung in unterschiedlichen Stellungen für einen komplexen Messablauf realisiert werden, wobei zu berücksichtigen ist, dass in einem kompakten, insbesondere tragbaren System nur ein Vorlaufantrieb und kein bidirektionaler Bandtransport mit vertretbarem Aufwand darstellbar ist. Zugleich ist durch die vorgegebene Abfolge der voneinander unterscheidbaren Positionsmarken eine Kontrollmöglichkeit gegen (ungewollte) Benutzermanipulation gegeben, so dass eine erhöhte

Testsicherheit gegeben ist und ein Verlust einzelner Testfelder oder des gesamten Testvorrats vermeidbar ist. Ein weiterer Vorteil ergibt sich dadurch, dass die Bereitstellungszeit für einen Test durch eine günstige Positionierung reduziert werden kann.

Vorteilhafterweise liefern die Positionsmarken aufgrund einer ungleichmäßigen Verteilung und/oder unterschiedlichen Abmessung und/oder unterschiedlichen Reflektivität als Messgröße beim Vorbeiführen an dem Bandsensor ein für ihre spezifische Abfolge eindeutiges Signalmuster.

Eine vorteilhafte Ausgestaltung sieht vor, dass die Funktionsstellungen zumindest eine Startposition und eine Messposition des auf dem jeweiligen Bandabschnitt befindlichen Testfelds definieren. Hierbei ist es günstig, wenn die Startpositionsmarke zum Positionieren des Testfelds in einer Startposition in einem gegebenen Abstand zu dem Testfeld angeordnet ist, so dass sich das Testfeld in der Startposition in einem gegenüber der Umgebung abgedichteten Vorratsbereich befindet. Durch eine solche zusätzliche Start- bzw. Ruheposition kann neben der geeigneten Lagerung des empfindlichen Testmaterials auch eine günstige Bandstellung eingenommen werden. Beispielsweise sollte der jeweils nächste Test ohne lange Verzögerung für den Anwender bereitstehen, wobei auch ungünstige Testfeldpositionen im Bereich von Dichtungen oder Umlenkstellen zu vermeiden sind.

Gemäß einer weiteren vorteilhaften Ausgestaltung besitzt jeder Bandabschnitt eine Messpositionsmarke zum Positionieren des Testfelds in einer Messposition, wobei das Testfeld in der Messposition mit Körperflüssigkeit beaufschlagbar oder beaufschlagt ist.

Um die Messgenauigkeit weiter verbessern zu können, ist mindestens eine Kontrollpositionsmarke zum Positionieren eines zugeordneten Kontrollfelds auf jedem Bandabschnitt vorgesehen, so dass das Kontrollfeld durch eine zur Vermessung des Testfelds vorgesehene Messeinheit abtastbar ist. Dabei sollte die Größe des Kontrollfelds so bemessen sein, dass ein von der Messeinheit erfasstes Messfenster vollständig überdeckbar ist und das Eindringen von Fremdlicht vermieden wird.

Eine weitere Verbesserung wird dadurch erreicht, dass mindestens eine Positionsmarke eine Zusatzfunktion als Kontrollfeld für eine Messeinheit aufweist, so dass auch die erforderliche Länge der mit einem Testfeld versehenen Bandabschnitte begrenzt werden kann.

Um die Fehlererkennung zu verbessern, ist mindestens eine vorzugsweise aus mehreren Einzelfeldern bestehende Steuerpositionsmarke vorgesehen.

Vorteilhafterweise besteht das Trägerband aus einem transparenten Folienmaterial, während die Positionsmarken durch optisch erfassbare Felder, insbesondere aufgedruckte Farbfelder auf dem Folienmaterial gebildet sein können.

Um die Bandcodestruktur eindeutig zu erfassen, sollte der von dem Bandsensor auflösbare Messfleck kleiner als die kleinste Positionsmarke sein.

Gegenstand der Erfindung ist auch ein Testbandgerät mit einem Bandantrieb für die als Verbrauchseinheit vorzugsweise in Form einer Bandkassette einwechselbare Testbandeinheit, einer Messeinheit zum Abtasten der Messfelder in einer Messposition und einer den Bandsensor umfassenden und mit einer Steuerungssoftware versehene Steuereinrichtung zum Ansteuern des Bandantriebs nach Maßgabe der Positionsmarken.

Um Signalschwankungen durch Lageänderungen und Materialeinflüsse des Bandmaterials möglichst zu vermeiden, sollte der Bandsensor eine Lichtquelle und einen Lichtempfänger aufweisen, wobei die Lichtquelle und der Lichtempfänger so auf das Trägerband ausgerichtet sind, dass im Wesentlichen nur diffus reflektiertes Licht erfasst wird. Herstellungstechnisch ist es auch günstig, wenn ein solcher Sensor als Baueinheit einseitig an dem Bandtransportweg montierbar ist.

Gemäß einer weiteren vorteilhaften Ausgestaltung ist die Steuereinrichtung dazu ausgebildet, in einer Lernphase beim Abtasten des Trägerbandes zughörige Signalpegel zu erfassen und daraus Schaltschwellen zur nachfolgenden Unterscheidung von Positionsmarken und anderen Bandbereichen zu ermitteln. Daraus ergibt sich eine besondere Robustheit der Bandpositionierung auch bei möglichen geräte- und bandseitigen Herstellungstoleranzen oder Alterungseffekten.

Ein weiterer Erfindungsaspekt liegt darin, dass die Steuereinrichtung zum Vergleich eines beim Vorpulen abgetasteten Signalmusters mit einem gespeicherten Signalmuster der Positionsmarken ausgebildet ist, so dass bei einer als Fehlerfall erkannten Abweichung ein nachfolgender Bandabschnitt bereitstellbar ist. Als Fehlerfall denkbar ist beispielsweise eine Anwender-Manipulation der Bandkassette außerhalb des Geräts, etwa durch manuelles Drehen der Aufwickelspule, so dass sich das Band beim Start nicht in einer regulären Position befindet.

Aus konstruktiven Gründen kann es von Vorteil sein, wenn der Bandsensor in einem vorgegebenen Abstand entlang des Bandtransportwegs vor oder nach der Messeinheit bzw. der Messposition gerätefest angeordnet ist. Grundsätzlich ist es aber auch möglich, dass der Bandsensor zugleich als Detektor für die Auswertung der Testfelder ausgebildet ist.

Im Folgenden wird die Erfindung anhand eines in der Zeichnung dargestellten Ausführungsbeispiels näher erläutert. Es zeigen:
- Fig. 1: ein analytisches Testbandgerät in einer schematischen Darstellung;
- Fig. 2: einen Bandabschnitt eines Testbands in zwei verschiedenen Funktionsstellungen; und
- Fig. 3: einen beim Abtasten eines Bandabschnitts des Testbands mittels eines Bandsensors erfassten Signalverlauf.

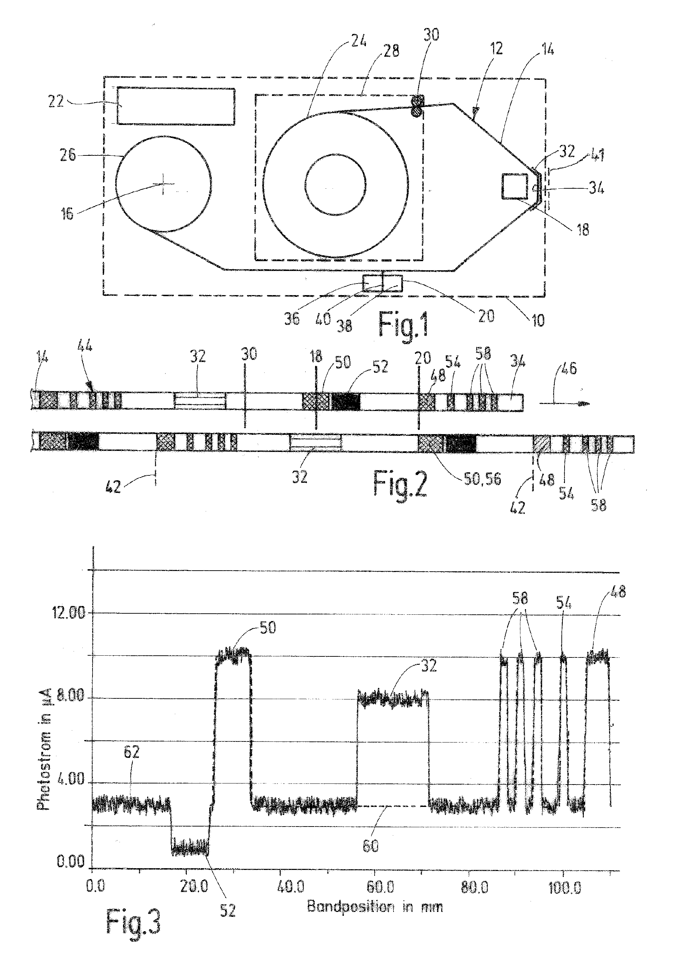

Das in Fig. 1 veranschaulichte tragbare Testbandgerät 10 ermöglicht als tragbares Analysesystem den Einsatz einer Testbandeinheit 12 mit vorspulbarem Testband 14 in Form einer Bandkassette zur Durchführung von Glukosetests. Das generelle Geräteprinzip ist in der EP-Anmeldung Nr. 02026242.4 beschrieben, worauf hier Bezug genommen wird. Um eine genaue Bandpositionierung für alle Funktionalitäten sicherstellen zu können, ist das Testband 14 mit einem speziellen Positioniercode versehen.

Das Testbandgerät 10 besitzt einen Bandantrieb 16, eine Messeinheit 18, einen Bandsensor 20 und eine mikroprozessorgestützte Steuereinrichtung 22. Der Bandsensor 20 ist der Messeinheit 18 in einem vorgegebenen Abstand bzw. Weg-Offset entlang des Bandtransportwegs nachgeordnet. Er kann grundsätzlich auch vorgeordnet sein.

Die Testbandeinheit 12 umfasst eine Vorratsspule 24 für unverbrauchtes Testband 14 und eine mit dem Antrieb 16 koppelbare Aufwickelspule 26 für verbrauchtes Testband. Die Vorratsspule 24 ist in einer Vorratskammer 28 angeordnet, die auch im Bereich des Banddurchzugs mittels Dichtung 30 nach außen abgedichtet ist.

Das Testband 14 ist abschnittsweise mit Testfeldern 32 versehen, die im Bereich einer Umlenkspitze vorderseitig mit Probenflüssigkeit, insbesondere Blut oder Gewebeflüssigkeit beaufschlagt werden können. Der Nachweis des Analyten (Glukose) erfolgt durch rückseitige reflexionsphotometrische Messung mittels der Messeinheit 18. Zu diesem Zweck umfasst das Testband 14 ein transparentes Träger- bzw. Transportband 34, auf dem die Testfelder 32 als Trockenreagenzschicht aufgebracht sind. Durch entsprechenden Bandvorlauf können die Testfelder 32 sukzessive zum Einsatz gebracht werden. Auf diese Weise lassen sich Vielfachtests für eine Patienten-Selbstkontrolle durchführen, ohne dass Verbrauchsmittel ausgetauscht werden müssten.

Der Bandsensor 20 ermöglicht eine sensorische Erfassung des Positioniercodes, so dass der Bandantrieb zur definierten Bandpositionierung entsprechend angesteuert werden kann. Der als Baueinheit seitlich an der Bandführung gerätefest angeordnete Bandsensor 20 weist eine LED als Lichtquelle 36 und einen Photodetektor 38 auf, wobei eine Abschirmung 40 ein direktes Lichtübersprechen verhindert. Durch eine geeignete Ausrichtung von Lichtquelle und Photodetektor außerhalb des spiegelnden Reflexionspfades wird im Wesentlichen nur das vom Testband 14 diffus zurückgeworfene Licht erfasst, so dass unabhängig von den Glanzeigenschaften und Lageveränderungen des hochflexiblen Testbands 14 eine robuste, zuverlässige Erkennung gewährleistet ist.

Eine schließbare Schutzabdeckung 41 schützt das Testband 14 im Bereich der Umlenkspitze bei Nichtgebrauch. Zur Handhabungsvereinfachung kann das Gerät durch Öffnen dieser beispielsweise schwenkbaren Schutzabdeckung 41 direkt eingeschaltet bzw. aktiviert werden, ohne dass noch zusätzliche Bedienschritte erforderlich wären.

Wie in Fig. 2 gezeigt, befinden sich die voneinander beabstandeten Testfelder 32 einzeln auf jeweils einem zugeordneten Bandabschnitt 42, der zusätzlich mit einem Positioniercode 44 in Form von verschiedenen Positionsmarken versehen ist. Beim Bandvorlauf (Pfeil 46) werden die Positionsmarken 44 relativ zu den gerätefesten Positionen von Dichtung 30, Messeinheit 18 und Bandsensor 20 bewegt.

Eine Startposition bzw. Parkposition (Fig. 2 oben) wird durch eine Startpositionsmarke 48 an der Erfassungsstelle des Bandsensors 20 definiert. In der Startposition befindet sich das nächste unverbrauchte Testfeld 32 in der gegenüber der Umgebung abgeschirmten Vorratskammer 28 in Wartestellung. Dabei liegt nur das dünne Trägerbandmaterial in der Dichtung 30, so dass diese dicht abschließt.

Es versteht sich, dass der Benutzer an einer schnellen Verfügbarkeit des Systems interessiert ist. Daher sollte die Bereitstellungszeit von der Geräteaktivierung bis zum Zeitpunkt der möglichen Blutapplikation in einem Bereich von wenigen Sekunden liegen. Entsprechend sollte der erforderliche Bandtransportweg, also der Weg zwischen der Dichtung 30 und dem Messort an der Umlenkspitze durch eine geeignete Gerätearchitektur möglichst kurz gehalten wird. Insbesondere sollte die Vorratskammer 28 nahe der Umlenkspitze angeordnet sein, und die Dichtung 30 sollte sich in Bandlaufrichtung gesehen möglichst weit vorne befinden. Die Bandlaufgeschwindigkeit sollte in einem Bereich von 10 bis 20 mm/s liegen, während die Distanz zwischen Dichtung 30 und Umlenkspitze ca. 25 mm und zwischen Umlenkspitze und Bandsensor ca. 30 mm beträgt.

Auch die Abfolge der Positionsmarken 44 ist zweckmäßig so angepasst, dass die Bereitstellungszeit möglichst verkürzt wird. Demgemäß sollten Funktions- bzw. Hardwaretests in dieser Phase auf einen hinreichenden Mindestumfang reduziert werden. Solche Tests können auch die Kontrolle des Verbrauchsmittelzustandes durch geeignete Kontrollfelder auf dem Trägerband umfassen.

In der Startposition befindet sich ein weißes Kontrollfeld 50 an der Umlenkspitze vor der Messeinheit 18. Das ebenso wie die anderen Positionsmarken auf das Trägerband 34 aufgedruckte Kontrollfeld 50 ist so bemessen, dass das von der Messeinheit 18 erfasste Messfenster vollständig überdeckt wird. In gleicher Weise kann auch ein vorgeordnetes schwarzes Kontrollfeld 52 mittels der zugeordneten Kontrollpositionsmarke 54 vor Einnahme der Parkposition zur Vermessung positioniert werden.

In der in Fig. 2 unten gezeigten Messposition befindet sich das Testfeld 32 entsprechend Fig. 1 vor der Messeinheit 18, während das weiße Kontrollfeld zugleich als Messpositionsmarke 56 an der Erfassungsstelle des Bandsensors 20 steht.

Als zusätzliche Steuerpositionsmarke sind drei Einzelfelder 58 vorgesehen, die aufgrund der beiden transparenten Zwischenräume deutlich von einem mit Blut beaufschlagten Testfeld 32 unterscheidbar sind.

Der vorstehend beschriebene Bandcode 44 wiederholt sich auf jedem mit einem Testfeld 32 versehenen Bandabschnitt 42, so dass bei gegebener Abschnittslänge beispielsweise 50 Tests in einer Bandkassette bevorratet werden können.

Die Positionsmarken 44 sind aufgrund ihrer ungleichmäßigen Verteilung und Größe beim Abtasten mittels des Bandsensors 20 identifizierbar bzw. voneinander unterscheidbar. Dementsprechend können verschiedene Funktionsstellungen wie Parken (Standby), Kontrollieren/Kalibrieren und Messen über die Steuereinheit 22 angesteuert werden. Zusätzlich ist es möglich, ungewollte Veränderungen der Bandstellung zu erkennen, wie es weiter unten beschrieben wird.

Fig. 3 zeigt das beim Bandvorlauf durch den Bandsensor 20 erfasste Signalmuster zusammen mit dem den Positionsmarken zugeordneten idealen Signalverlauf (gestrichelte Linie 60). In dem Messdiagramm ist der Photostrom des Bandsensors 20 als Messgröße über der Bandposition bzw. dem Bandvorschub aufgetragen (die Länge eines Bandabschnitts 42 beträgt hier 110 mm). Ausgehend von der Startposition (vgl. Fig. 2 oben) wird zunächst das transparente Trägerband 34 vor dem Hintergrund des dunklen Geräteinneren als Signalpegel 62 erfasst. Darauf folgen die Signale der Positionsmarken und des Testfelds in den gegebenen Abständen und Abmessungen. Um auch die Steuerpositionsmarke 58 eindeutig erfassen zu können, muss gewährleistet sein, dass die beim Bandtransport abgetastete Dimension der schmalen Einzelfelder und Zwischenräume nicht die Auflösung des Bandsensors 20 unter Berücksichtigung aller Toleranzen unterschreitet. Hierbei gehen auch die Abtastrate sowie Trägheit des Bandsensors und die Bandgeschwindigkeit ein.

In der Ablaufsteuerung ist ein Pegel-Lernen für den Bandsensor 20 implementiert. Hierbei wird in einer Lernphase zu Beginn der Bereitstellung des Testfelds 32 sowohl ein Dunkelfeld- als auch ein Hellfeld-Signalpegel aufgenommen und für die Dauer eines Tests gespeichert. Insbesondere kann als Dunkelfeld auch der Signalpegel 62 des transparenten Trägerbands 34 vor der schwarzen Gehäuseinnenwand erfasst werden. In der Regel werden jedoch das schwarze und weiße Kontrollfeld 52, 50 herangezogen. Bezüglich dieser Signalpegel werden dann Schaltschwellen für den Hell/Dunkel-Übergang definiert, die eine zuverlässige Unterscheidung zwischen den Positionsmarken 44 und allen anderen Bereichen des Testbandes 14 gewährleisten. Somit ist es möglich, auch bei etwaigen Produktionsschwankungen oder Alterungsprozessen eine exakte Positionierung sicherzustellen.

Die Ablaufsteuerung ist dazu ausgebildet, den Bandtransport anhand der sequentiellen Abfolge der Positionsmarken 44 zu überprüfen und bei eventuellen Manipulationen einen größeren Testmaterialverlust möglichst zu vermeiden. Auf diese Weise können auch Fehlerfälle erkannt werden, die auf einer Benutzermanipulation der Bandkassette beruhen. Ein solcher Fehlerfall ist beispielsweise dadurch gegeben, dass die Kassette so weitergedreht wurde, dass sich das weiße Kontrollfeld 50 im Bereich der Messposition befindet. Wegen der Unmöglichkeit des Einlernens des Grundpegels wird der Antrieb daraufhin unterbrochen. Die Steuereinheit 22 registriert einen verlorenen Test und fährt das nächste weiße Kontrollfeld 50 vor die Messeinheit 18. Anschließend wird das Testfeld 32 an der Messstelle positioniert, und die Probenflüssigkeit kann aufgetragen werden. Hierbei wird im Testfeldaufbau durch eine Spreitschicht in der Regel sichergestellt, dass sich nur ein einzelner zusammenhängender Probenfleck ausbildet. Das Signal der zweifach unterbrochenen Steuerpositionsmarke 58 kann davon deutlich unterschieden werden, so dass auch der Fall eines manipulierten Weiterspulens bis in den Bereich hinter das Testfeld 32 eindeutig erfassbar wäre. Abschließend wird durch die Positionsmarke 54 das Ausmessen des schwarzen Kontrollfelds 52 ermöglicht. Durch das Fehlerhandling wird in jedem Fall gewährleistet, dass bei einer Fehlpositionierung nicht die ganze Bandkassette verworfen werden muss.

## Patentansprüche

1. Testbandeinheit mit einem durch einen Bandantrieb (16) vorspulbaren flexiblen Trägerband (34), auf welchem eine Vielzahl von analytischen Testfeldern (32) zur Beaufschlagung mit Körperflüssigkeit insbesondere für Glukosetests aufgebracht sind, wobei sich jedes Testfeld (32) auf einem zugehörigen Bandabschnitt (42) befindet, **dadurch gekennzeichnet, dass** jeder Bandabschnitt (42) mehrere mittels eines Bandsensors (20) abtastbare und dabei durch eine Messgröße einzeln identifizierbare Positionsmarken (44) für unterschiedliche Funktionsstellungen aufweist.

2. Testbandeinheit nach Anspruch 1, **dadurch gekennzeichnet, dass** die Positionsmarken (44) durch eine ungleichmäßige Verteilung und/oder unterschiedliche Abmessung und/oder unterschiedliche Reflektivität beim Vorbeiführen an dem Bandsensor (20) ein für ihre Abfolge eindeutiges Signalmuster liefern.

3. Testbandeinheit nach Anspruch 1 oder 2, **dadurch gekennzeichnet, dass** die Funktionsstellungen zumindest eine Startposition und eine Messposition des auf dem jeweiligen Bandabschnitt (42) befindlichen Testfelds (32) definieren.

4. Testbandeinheit nach einem der Ansprüche 1 bis 3,
**dadurch gekennzeichnet, dass** eine Startpositionsmarke (48) zum Positionieren des Testfelds (32) in einer Startposition in einem gegebenen Abstand zu dem Testfeld (32) angeordnet ist, wobei sich das Testfeld (32) in der Startposition in einem gegenüber der Umgebung abgedichteten Vorratsbereich (28) befindet.

5. Testbandeinheit nach einem der Ansprüche 1 bis 4,
**dadurch gekennzeichnet, dass** jeder Bandabschnitt (42) eine Messpositionsmarke (56) zum Positionieren des Testfelds (32) in einer Messposition aufweist, wobei das Testfeld (32) in der Messposition mit Körperflüssigkeit beaufschlagbar oder beaufschlagt ist.

6. Testbandeinheit nach einem der Ansprüche 1 bis 5,
**dadurch gekennzeichnet, dass** mindestens eine Kontrollpositionsmarke (54) zum Positionieren eines zugeordneten Kontrollfelds (52) auf jedem Bandabschnitt (42) angeordnet ist, so dass das Kontrollfeld (52) durch eine zur Vermessung des Testfelds (32) vorgesehene Messeinheit (18) abtastbar ist.

7. Testbandeinheit nach Anspruch 6, **dadurch gekennzeichnet, dass** die Größe des Kontrollfelds (52) so bemessen ist, dass ein von der Messeinheit (18) erfasstes Messfenster vollständig überdeckbar ist.

8. Testbandeinheit nach einem der Ansprüche 1 bis 7,
**dadurch gekennzeichnet, dass** mindestens eine Positionsmarke (44) eine Zusatzfunktion als Kontrollfeld (50) für eine Messeinheit (18) aufweist.

9. Testbandeinheit nach einem der Ansprüche 1 bis 8,
**gekennzeichnet durch** mindestens eine vorzugsweise aus mehreren Einzelfeldern bestehende Steuerpositionsmarke (58) zur Ablaufsteuerung des Bandvorlaufs.

10. Testbandeinheit nach einem der Ansprüche 1 bis 9,
**dadurch gekennzeichnet, dass** das Trägerband (34) aus einem transparenten Folienmaterial besteht, und dass die Positionsmarken (44) durch optisch erfassbare Felder, insbesondere aufgedruckte oder aufgeklebte Farbfelder auf dem Folienmaterial gebildet sind.

11. Testbandeinheit nach einem der Ansprüche 1 bis 10,
**dadurch gekennzeichnet, dass** der von dem Bandsensor (20) auflösbare Messfleck kleiner als die kleinste Positionsmarke (58) ist.

12. Testbandgerät zum Verarbeiten einer Testbandeinheit nach einem der vorhergehenden Ansprüche, mit einem Bandantrieb (16) für die als Verbrauchseinheit vorzugsweise in Form einer Bandkassette einwechselbare Testbandeinheit (12), einer Messeinheit (18) zum Abtasten der Testfelder (32) in einer Messposition und einer einen Bandsensor (20) umfassenden Steuereinrichtung (22) zum Ansteuern des Bandantriebs (16) nach Maßgabe der Positionsmarken (44).

13. Testbandgerät nach Anspruch 12, **dadurch gekennzeichnet, dass** der Bandsensor (20) eine Lichtquelle (36) und einen Lichtempfänger (38) aufweist, wobei die Lichtquelle und der Lichtempfänger so auf das Trägerband (34) ausgerichtet sind, dass im Wesentlichen nur diffus reflektiertes Licht erfasst wird.

14. Testbandgerät nach Anspruch 12 oder 13, **dadurch gekennzeichnet, dass** die Steuereinrichtung (22) dazu ausgebildet ist, in einer Lernphase beim Abtasten des Trägerbands (34) zughörige Signalpegel zu erfassen und daraus Schaltschwellen zur nachfolgenden Unterscheidung von Positionsmarken (44) und anderen Bandbereichen zu ermitteln.

15. Testbandgerät nach einem der Ansprüche 12 bis 14,
**dadurch gekennzeichnet, dass** die Steuereinrichtung (22) zum Vergleich eines beim Vorpulen abgetasteten Signalmusters mit einem gespeicherten Signalmuster der Positionsmarken (44) ausgebildet ist, so dass bei einer als Fehlerfall erkannten Abweichung ein nachfolgender Bandabschnitt (42) bereitstellbar ist.

16. Testbandgerät nach einem der Ansprüche 12 bis 15, **dadurch gekennzeichnet, dass** der die Positionsmarken erfassende Bandsensor (20) in einem vorgegebenen Abstand entlang des Bandtransportwegs vor oder nach der Messeinheit (18) gerätefest angeordnet ist.

17. Testbandeinheit nach einem der Ansprüche 12 bis 16, **dadurch gekennzeichnet, dass** in einer Startposition ein Kontrollfeld (50) für eine Funktionskontrolle vor der Messeinheit (18) angeordnet ist, wobei der Testbandbereich zwischen dem Kontrollfeld (50) und dem nächsten unverbrauchten Testfeld (32) frei von Positionsmarken (44) ist.

18. Testbandgerät nach einem der Ansprüche 12 bis 17, **dadurch gekennzeichnet, dass** die Bereitstellungszeit zwischen dem Einschalten bzw. der Geräteaktivierung und der Bereitstellung eines Testfelds (32) an der Messposition kleiner als 8 s, vorzugsweise kleiner als 3 s ist.

19. Testbandgerät nach einem der Ansprüche 12 bis 18, **dadurch gekennzeichnet, dass** eine Schutzabdeckung für das Testband beim Öffnen zugleich einen Schalter zum direkten Einschalten des Geräts bildet.
